# EUROPEAN PATENT APPLICATION

(11) **EP 3 636 673 A1**
(43) Date of publication of application: **15.04.2020**
(21) Application number: 18814393.7
(22) Date of filing: 11.06.2018
(51) Int. Cl.: C08B 30/00, C08B 30/12, C08B 31/06, C12P 19/04, A21D 2/18

(54) **GELATINIZED STARCH**

(30) Priority: 09.06.2017 JP 2017114628
(71) Applicant: Glico Nutrition Co., Ltd., Osaka-shi, Osaka 555-8502 (JP)
(72) Inventor: KITAAKI, Kohei, Osaka-shi Osaka 555-8502 (JP); HIROSE, Tahiro, Osaka-shi Osaka 555-8502 (JP); YODA, Yasuhiro, Osaka-shi Osaka 555-8502 (JP); KURITA, Kenichi, Osaka-shi Osaka 555-8502 (JP)
(74) Representative: Uexküll & Stolberg
(86) International application number: PCT/JP2018/022239
(87) International publication number: WO 2018/225872

(57) **Abstract**

The purpose of the present invention is to provide: a pregelatinized starch which, upon addition of water thereto, can be rapidly dispersed while being inhibited from forming lumps and give a dispersion having a sufficient viscosity; and a method for producing the pregelatinized starch. The pregelatinized starch has a degree of swelling with cold water of 7-10. The pregelatinized starch, when examined by a given mixograph test method, gives a middle line (moving average line) for torque (torque%) and satisfies Y(t)≤Y(t+0.1) where t is the time (sec) which has passed since measurement initiation and is any of the range of 0 to 99.9 seconds, Y(t) is the torque (torque%) at after t seconds determined from the middle line, and Y(t+0.1) is the consistency (torque%) at after (t+0.1) seconds determined from the middle line.

## Description

### TECHNICAL FIELD

The present invention relates to a pregelatinized starch which, upon addition of water thereto, can be rapidly dispersed while suppressing formation of lumps and give a dispersion having a sufficient viscosity. The present invention also relates to a method for producing the pregelatinized starch.

### BACKGROUND ART

Since a pregelatinized starch swells upon addition of water thereto, even under conditions where heat is not applied or under conditions where starch is not gelatinized due to low water content, the pregelatinized starch can impart viscosity and express gel properties like starch after gelatinization. Therefore, the pregelatinized starch is used in various foods.

On the other hand, the pregelatinized starch generally has a remarkably high water absorption rate, so that when the pregelatinized starch is dispersed or dissolved in water, lumps (masses of the pregelatinized starch) tend to be formed, and there is a disadvantage that operability during food production is poor. Thus, techniques for improving dispersibility of the pregelatinized starch in water have been studied.

For example, Patent Document 1 reports that formation of lumps can be suppressed by an alkali-soluble pregelatinized starch having a solubility in water of 70% by weight or less at 20°C when pH is 6 or less and having a solubility in water of 90% by weight or more when pH is 12 or more. However, the technique of Patent Document 1 assumes that the alkali-soluble pregelatinized starch is used by being dissolved in an alkaline aqueous solution. It is difficult to use the alkali-soluble pregelatinized starch in the food field, and a pregelatinized starch is in a completely dissolved state; therefore, there is a disadvantage that original viscosity of the pregelatinized starch cannot be sufficiently imparted due to disintegration of starch granules.

Patent Document 2 reports that 0.1 to 10% of an emulsifier per starch is mixed with a starch slurry and dried by drum dryer to obtain a pregelatinized starch excellent in dispersibility. However, the technique of Patent Document 2 uses the emulsifier as a secondary material and has a disadvantage that when the pregelatinized starch is used in food, the food may be affected by the emulsifier.

Furthermore, Patent Document 3 reports that the dispersibility of a pregelatinized starch can be improved by blending oils and fats in a proportion of 3 to 19% by weight. However, even in the technique of Patent Document 3, since the use of secondary materials (oils and fats) is indispensable, the pregelatinized starch cannot be applied to foods containing no fat and oil, and there is a disadvantage that the types of usable foods are limited.

As described above, in the prior art, the pregelatinized starch which improves dispersibility and reduces possibility of formation of lumps is not fully satisfactory assuming the use in the food field. If properties of the pregelatinized starch itself are modified to reduce possibility of formation of lumps, viscosity that can be imparted tends to decrease. For this reason, in the prior art, currently, the modification of the properties of the pregelatinized starch itself has not been able to achieve both suppression of formation of lumps and expression of sufficient viscosity.

### PRIOR ART DOCUMENTS

### PATENT DOCUMENTS

Patent Document 1: Japanese Patent Laid-Open No. H5-155901
Patent Document 2: Japanese Patent Laid-Open No. 2000-38401
Patent Document 3: Japanese Patent Laid-Open No. H8-154606

### SUMMARY OF THE INVENTION

### PROBLEMS TO BE SOLVED BY THE INVENTION

An object of the present invention is to provide a pregelatinized starch which, upon addition of water thereto, can be rapidly dispersed while suppressing formation of lumps and give a dispersion having a sufficient viscosity, and a method for producing the pregelatinized starch.

### MEANS FOR SOLVING THE PROBLEM

As a result of diligent studies to solve the above problems, the present inventors have found that a pregelatinized starch obtained by applying predetermined crosslinking treatment and enzymatic treatment to starch and then pregelatinizing the starch by drum dryer is excellent in dispersibility in water, and even when water is added, it is possible to suppress formation of lumps and, in addition, to provide a sufficient viscosity.

The present inventors have found that the pregelatinized starch satisfies the following specific characteristics (i) and (ii):
(i) A degree of cold water swelling is 7 to 10; and
(ii) A middle line (moving average line) for torque (torque%) is determined by a given mixograph test method, and when t is a time (sec) which has passed since measurement initiation, Y(t) is a torque (torque%) after t seconds determined from the middle line, and Y(t +0.1) is a consistency (torque%) after t + 0.1 seconds determined from the middle line, Y(t) ≤ Y(t + 0.1) is satisfied in all range where t is 0 to 99.9 seconds.

The present invention has been completed as a result of further study based on these findings. In other words, the present invention provides the inventions represented by the following aspects.

Item 1. A pregelatinized starch having a degree of cold water swelling of 7 to 10,
a middle line (moving average line) for torque (torque%) being determined by the following mixograph test method, and when t is a time (sec) which has passed since measurement initiation, Y(t) is a torque (torque%) after t seconds determined from the middle line, and Y(t + 0.1) is a consistency (torque%) after t + 0.1 seconds determined from the middle line, Y(t) ≤ Y(t + 0.1) is satisfied in all range where t is 0 to 99.9 seconds:

### <Mixograph test method>

25 ml of water is added to a mixture of 5 g of target pregelatinized starch and 25 g of strong flour, and according to AACC (American Association of Cereal Chemists) method 54-40.02 established by AACC, a change in torque (torque%) is measured for 100 seconds at a temperature of 15°C by a mixograph test method.

Item 2. The pregelatinized starch according to Item 1, in which a viscosity measured under the following conditions is 1000 mPa·s or more:

### <Conditions for measuring viscosity>

While 180 ml of water at 25°C contained in a 200 ml beaker is stirred at 1200 rpm with a stirrer, 1 g in terms of dry weight of a pregelatinized starch to be measured is quickly added and stirred for 1 minute; thereafter, using a B-type viscometer (VISCOMETER TV-20, manufactured by Toki Sangyo Co., Ltd.), viscosity is measured under conditions where a No. 5 rotor, 25°C, rotation speed of 30 rpm, and after an elapse of 1 minute.

Item 3. The pregelatinized starch according to Item 1 or 2, which is a modified pregelatinized starch subjected to a crosslinking treatment and an enzymatic treatment.

Item 4. A method for producing a modified pregelatinized starch starch including
a step 1 of applying a crosslinking treatment and an enzymatic treatment to a starch to obtain an enzyme-treated crosslinked starch , and
a step 2 of applying a pregelatinizing treatment to the enzyme-treated crosslinked starch obtained in the step 1 to obtain a pregelatinized enzyme-treated crosslinked starch.

Item 5. The method for producing a pregelatinized starch according to Item 4, in which the crosslinking treatment in the step 1 is a phosphate-crosslinking treatment.

Item 6. The method for producing a pregelatinized starch according to Item 4 or 5, in which the enzymatic treatment in the step 1 is a treatment using an amylolytic enzyme.

Item 7. The method for producing a pregelatinized starch according to any one of Items 4 to 6, in which the enzymatic treatment is applied after the crosslinking treatment is applied in the step 1.

Item 8. The method for producing a pregelatinized starch according to any one of Items 4 to 7, in which the pregelatinizing treatment in the step 2 is a pregelatinization treatment using a drum dryer.

### EFFECTS OF THE INVENTION

Since the pregelatinized starch of the present invention can be rapidly dispersed while suppressing formation of lumps when added to water and provides a sufficient viscosity, operability during production of processed foods using a pregelatinized starch can be improved.

For example, when the pregelatinized starch of the present invention is added to dough of bakery products, sufficient formation of gluten in a wheat flour is promoted during production. Even if the amount of water added is increased, the dough is not sticky, and workability can be improved. Furthermore, in bakery products blended with the pregelatinized starch of the present invention, the pregelatinized starch of the present invention gives a sufficient viscosity, so that the bakery products have water retention and can have a fluffy and soft texture.

For example, when the pregelatinized starch of the present invention is blended in kneaded dough of fish paste products, sufficient viscosity is given by the pregelatinized starch of the present invention, so that even if the amount of fish meat used is reduced by about 10 to 30%, the texture of the fish paste product to be produced is not affected, and the kneaded dough can be provided with a suitable viscosity. In the fish paste products blended with the pregelatinized starch of the present invention, even after heating, the food texture with elasticity similar to fish meats can be imparted.

### BRIEF DESCRIPTION OF THE DRAWINGS

Fig. 1 is a graph showing a middle line for torque (torque%) determined by a mixograph test method for pregelatinized starches of Examples 1 and 2.
Fig. 2 is a graph showing the middle line for the torque (torque%) determined by the mixograph test method for pregelatinized starches of Comparative Examples 1 to 4.

### EMBODIMENTS OF THE INVENTION

A pregelatinized starch of the present invention has a degree of cold water swelling of 7 to 10, and in a predetermined mixograph test, when t is a time (sec) which has passed since measurement initiation, Y(t) is a consistency (torque%) after t seconds from the measurement initiation, and Y(t + 0.1) is a consistency (torque%) after t + 0.1 seconds from the measurement initiation, Y(t) ≤ Y(t + 0.1) is satisfied in all range where t is 0 to 99.9 seconds. The pregelatinized starch of the present invention will be hereinafter described in detail.

### Degree of cold water swelling

The pregelatinized starch of the present invention has a degree of cold water swelling of 7 to 10. Satisfaction of such a degree of cold water swelling and mixograph characteristics described later can serve to achieve both suppression of formation of lumps and expression of sufficient viscosity. From the standpoint of more effectively achieving both suppression of formation of lumps and expression of sufficient viscosity, the degree of cold water swelling is preferably 7.5 to 9.5, more preferably 7 to 9.

In the present invention, the degree of cold water swelling is a value measured according to the following method.

### <Method for measuring degree of cold water swelling>

1 g in terms of dry weight of a pregelatinized starch to be measured is dispersed in 100 ml of water at 25°C contained in a 200 ml beaker and is swollen by stirring for 30 minutes at 25°C. Thereafter, centrifugation (300 rpm, for 10 minutes) to separate the dispersion into a gel layer and a supernatant layer. The weight of the gel layer is then measured and taken as A. The gel layer whose weight has been measured is thereafter dried to a solid state (of constant weight at 105°C), and the weight of the resulting solid is measured and taken as B. A value of A/B is calculated as the degree of cold water swelling.

### Mixograph characteristics

In the pregelatinized starch of the present invention, a middle line (moving average line) for torque (torque%) is determined by the following mixograph test method, and when t is the time (sec) which has passed since measurement initiation, Y(t) is the torque (torque%) after t seconds determined from the middle line, and Y(t + 0.1) is the torque (torque%) after t + 0.1 seconds determined from the middle line, Y(t) ≤ Y(t + 0.1) is satisfied in all range where t is 0 to 99.9 seconds.

### <Mixograph test method>

25 ml of water is added to a mixture of 5 g of target pregelatinized starch and 25 g of strong flour, and according to AACC (American Association of Cereal Chemists) method 54-40.02 established by AACC, a change in torque (torque%) is measured for 100 seconds at a temperature of 15°C by a mixograph test method (35-g model).

In the mixograph test method, the value of the torque (torque%) has an amplitude at every measurement time. In the present invention, the middle line (moving average line) is calculated by averaging the amplitude of the torque (torque%) for each measurement time, and the values of Y(t) and Y(t + 0.1) are calculated from the middle line. The middle line can be obtained by using a known analysis means. Specifically, the middle line can be obtained by using software of MIX SMARTver2.0.590 (manufactured by National Cereal Chemistry Equipment) and setting Midcurve filter to 80, No.Stages to 2, Mid slope window (%) to 7.5, and Mid peak fit window (%) to 5. In the mixograph test method, the value of the torque (torque%) is 100% when the measured torque value reaches to the maximum limit.

The pregelatinized starch of the present invention has the degree of cold water swelling and such mixograph characteristics, so that it is possible to achieve both suppression of formation of lumps and expression of sufficient viscosity. On the other hand, in a pregelatinized starch in which t does not satisfy Y(t) ≤ Y(t + 0.1) in any of a range of 0 to 99.9 seconds, an increase in viscosity due to water absorption and a decrease in viscosity due to breakdown occur, and the water absorption rate is high. When this pregelatinized starch is added to water, formation of lumps cannot be suppressed.

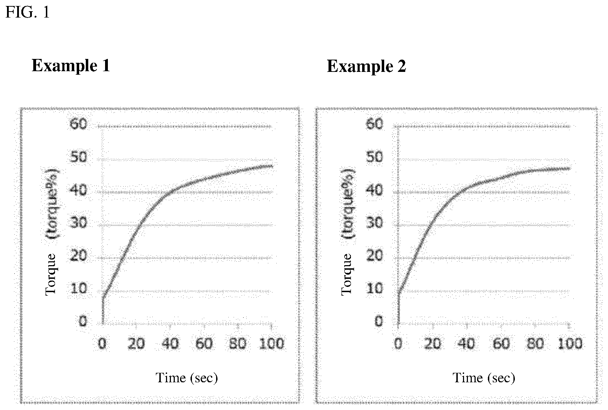

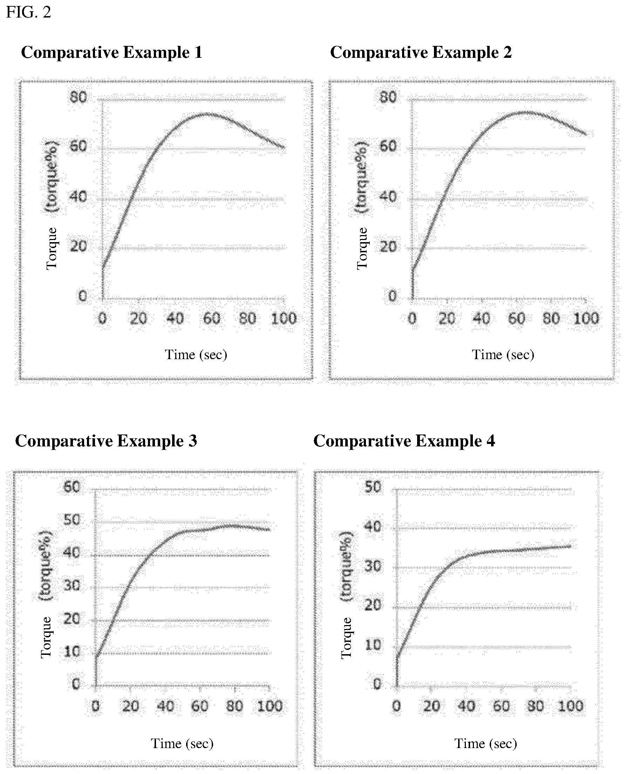

Such a characteristic that Y(t) ≤ Y(t + 0.1) is satisfied in all range where t is 0 to 99.9 seconds in the mixograph test means that no maximum peak is observed in the middle line in the range of t from 0 to 99.9 seconds. Specifically, in the pregelatinized starch satisfying Y(t) ≤ Y(t + 0.1) in all range where t is 0 to 99.9 seconds, as shown in the results of Examples 1 and 2 in Fig. 1, no maximum point is observed in the middle line. In the pregelatinized starch that may not satisfy Y(t) ≤ Y(t + 0.1) at any elapsed time in which t is 0 to 99.9 seconds, as shown in the results of Comparative Examples 1 to 3 in Fig. 2, the maximum point is observed in the middle line.

### Viscosity characteristics

The pregelatinized starch of the present invention can express sufficient viscosity upon addition of water thereto. As a suitable example of viscosity characteristics of the pregelatinized starch of the present invention, the viscosity measured under the following conditions is 1000 mPa·s or more, preferably 1000 to 20000 mPa·s, more preferably 1000 to 15000 mPa·s.

### <Conditions for measuring viscosity>

While 180 ml of water at 25°C contained in a 200 ml beaker is stirred at 1200 rpm with a stirrer, 1 g in terms of dry weight of a pregelatinized starch to be measured is quickly added and stirred for 1 minute. Thereafter, using a B-type viscometer (VISCOMETER TV-20, manufactured by Toki Sangyo Co., Ltd.), viscosity is measured under conditions where a No. 5 rotor, 25°C, rotation speed of 30 rpm, and after an elapse of 1 minute.

### Starch material

The type of starch material from which the pregelatinized starch of the present invention is derived is not particularly limited, and examples include tapioca starch, wheat starch, rice starch, glutinous rice starch, corn starch, waxy corn starch, sago starch, potato starch, mung bean starch, sweet potato starch, waxy potato starch, waxy tapioca starch, and waxy wheat starch. Among these starch materials, tapioca starch is preferably used from the viewpoint of suitably providing the degree of cold water swelling, mixograph characteristics and viscosity characteristics, and more favorably achieving both suppression of formation of lumps and expression of sufficient viscosity.

### Modified characteristics

Although unmodified pregelatinized starch cannot satisfy the above-described degree of cold water swelling and mixograph characteristics, when desired processing such as chemical treatment or enzymatic treatment is applied, the pregelatinized starch can satisfy the above-described degree of cold water swelling and mixograph characteristics.

The processing applied to the pregelatinized starch of the present invention is not particularly limited as long as the degree of cold water swelling and the mixograph characteristics can be satisfied, and from the viewpoint of suitably satisfying the degree of cold water swelling and the mixograph characteristics, preferable examples include a pregelatinized starch having been subjected to crosslinking treatment and enzymatic treatment (that is, pregelatinized enzyme-treated crosslinked starch).

The crosslinking treatment is a process of crosslinking hydroxyl groups at two or more locations in starch with a crosslinking agent. The type of crosslinking treatment used in the present invention is not particularly limited, and examples thereof include phosphate-crosslinking treatment and dicarboxylic acid crosslinking treatment.

The phosphate-crosslinking treatment is a process of crosslinking starch using a crosslinking agent such as trimetaphosphate or phosphorus oxychloride. Among the phosphate-crosslinking treatments, a crosslinking treatment using trimetaphosphate is preferable from the viewpoint of suitably satisfying the degree of cold water swelling and the mixograph characteristics.

Dicarboxylic acid-crosslinked starch treatment is a process of crosslinking starch using dicarboxylic acid such as adipic acid as a crosslinking agent.

Among these crosslinking treatments, from the viewpoint of suitably satisfying the degree of cold water swelling and mixograph characteristics and more favorably achieving both suppression of formation of lumps and expression of sufficient viscosity, phosphate-crosslinking treatment is preferable, and phosphate-crosslinking treatment using a trimetaphosphate is more preferable.

Enzymatic treatment is a process in which a part of starch is hydrolyzed by amylolytic enzyme or saccharide is transferred to starch by glycosyltransferase. The type of enzyme to be used is not particularly limited as long as the above-described degree of cold water swelling and mixograph characteristics can be satisfied, and examples include amylolytic enzymes such as amyloglucosidase, α-amylase, β-amylase, amyloglucosidase, isoamylase, and α-glucosidase; and glycosyltransferases such as cyclodextrin glucanotransferase. In the enzymatic treatment, one type of enzyme may be used alone, or two or more types of enzymes may be used in combination.

The sources of these enzymes are not particularly limited. In the case of amyloglucosidase, examples include Aspergillus niger, Rhizopus niveus, and Rhizopus oryzae. In the case of α-glucosidase, examples include Aspergillus niger and Aspergillus oryzae. In the case of isoamylase, examples include Pseudomonas amyloderamosa. In the case of cyclodextrin glucanotransferase, examples include Bacillus licheniformis and Paenibacillus macerans (Bacillus macerans).

Among these enzymes, from the viewpoint of suitably satisfying the degree of cold water swelling and the mixograph characteristics and more favorably achieving both suppression of formation of lumps and expression of sufficient viscosity, the amylolytic enzyme is preferably used, amyloglucosidase is more preferably used, and amyloglucosidase derived from Aspergillus niger is particularly preferably used.

The pregelatinized enzyme-treated crosslinked starch may be obtained by applying a crosslinking treatment to a starch material and then carring out an enzymatic treatment, or may be obtained by applying the enzymatic treatment to the starch material and then applying the crosslinking treatment. From the viewpoint of suitably satisfying the degree of cold water swelling and mixograph characteristics and more favorably achieving both suppression of formation of lumps and expression of sufficient viscosity, preferable examples include a pregelatinized enzyme-treated crosslinked starch obtained by applying the crosslinking treatment to a starch material and then carrying out the enzymatic treatment.

The pregelatinized enzyme-treated crosslinked starch may be subjected to other chemical treatment in addition to the crosslinking treatment and the enzymatic treatment t, as long as the degree of cold water swelling and the mixograph characteristics are satisfied. Examples of such chemical treatment include oxidation; esterification such as acetylation and phosphorylation; and etherification such as hydroxypropylation. One of these chemical treatments may be carried out alone, or two or more thereof may be carried out in combination. These chemical treatments can be carried out according to known methods.

### Production method

The method for producing the pregelatinized starch of the present invention is not particularly limited as long as the pregelatinized starch satisfying the degree of cold water swelling and the mixograph characteristics is obtained, and suitable examples include a production method including the following steps 1 and 2.

Step 1 of applying a crosslinking treatment and an enzymatic treatment to a starch to obtain an enzyme-treated crosslinked starch.

Step 2 of applying a pregelatinizing treatment to the enzyme-treated crosslinked starch obtained in the step 1 to obtain a pregelatinized enzyme-treated crosslinked starch.

Hereinafter, the steps 1 and 2 will be described for each step.

### [Step 1]

In the step 1, a starch is subjected to a crosslinking treatment and an enzymatic treatment. The order of the crosslinking treatment and the enzymatic treatment in the step 1 is not particularly limited, and the enzymatic treatment may be carried out after the crosslinking treatment, or the crosslinking treatment may be carried out after the enzymatic treatment. From the viewpoint of efficiently obtaining a pregelatinized starch suitably satisfying the degree of cold water swelling and mixograph characteristics and more favorably achieving both suppression of formation of lumps and expression of sufficient viscosity, in the step 1, it is preferable to carry out the enzymatic treatment after the crosslinking treatment.

The types of starch used in the step 1 are as described in the column "Starch material". The types of crosslinking treatment carried out in the step 1 are as described in the column "Modified characteristics".

The crosslinking treatment applied to the starch or the enzyme-treated starch can be carried out by a known method, and if the phosphate-crosslinking treatment using trimetaphosphate is carried out in the step 1, the following method may be mentioned.

First, a starch dispersion containing starch or enzyme-treated starch and an inorganic salt is provided. The type of inorganic salt is not particularly limited, and examples include chloride salts such as sodium chloride and calcium chloride; and inorganic acid salts such as sodium sulfate and calcium sulfate. Preferable examples include sodium chloride. The concentration of starch or enzyme-treated starch in the starch dispersion is not particularly limited, and is, for example, 10 to 45% by weight, preferably 20 to 45% by weight, more preferably 30 to 45% by weight. The concentration of inorganic salt in the starch dispersion is not particularly limited, and setting may be performed such that the amount of the inorganic salt is 0.5 to 15 parts by weight, preferably 1 to 10 parts by weight, more preferably 2 to 5 parts by weight per 100 parts by weight of starch.

pH of the starch dispersion may be set within a pH range in which phosphate-crosslinking reaction using trimetaphosphate can proceed. The pH of the starch dispersion is specifically 10.0 to 12.0, preferably 11.0 to 11.7, more preferably 11.3 to 11.5. The pH of the starch dispersion can be adjusted using an alkali such as sodium hydroxide or potassium hydroxide.

Next, trimetaphosphate (alkali metal salt such as sodium salt or potassium salt) is added to the starch dispersion to be reacted, and the starch is subjected to phosphate-crosslinking. The amount of trimetaphosphate added may be appropriately set in consideration of the degree of phosphate-crosslinking to be applied, and the like. For example, the amount of trimetaphosphate added may be set to 0.05 to 2.0 parts by weight, preferably 0.1 to 1.5 parts by weight, more preferably 0.2 to 1.0 parts by weight per 100 parts by weight of starch. The reaction temperature for the crosslinking reaction of trimetaphosphate may be appropriately set within a range in which the phosphate-crosslinking reaction can proceed, and is usually 30 to 50°C, preferably 35 to 45°C, more preferably 40 to 43°C. The reaction time for the crosslinking reaction of trimetaphosphate may be appropriately set according to the degree of phosphate-crosslinking to be applied, and the like, and is usually 1 to 10 hours, preferably 2 to 9 hours, more preferably 3 to 8 hours.

The degree of crosslinking of the starch after the crosslinking treatment is not particularly limited, but from the viewpoint of producing a pregelatinized starch having the degree of cold water swelling and the mixograph characteristics and more favorably achieving both suppression of formation of lumps and expression of sufficient viscosity, amylo viscosity in the following amylogram measurement satisfies 300 BU or less, preferably 10 to 300 BU, more preferably 10 to 200 BU. The amylo viscosity is a physical property value that serves as an index of the degree of crosslinking of crosslinked starch, and indicates that the lower the amylo viscosity, the higher the degree of crosslinking. The aforementioned range of the amylo viscosity can be satisfied by appropriately adjusting the amount of the crosslinking agent (trimetaphosphate, etc.) used, the time for the crosslinking reaction, and the like.

### (Conditions for amylogram measurement)

A slurry is prepared by adding water to a starch after the crosslinking treatment to be measured to adjust the starch concentration to 10% by weight. The measurement is started at 50°C, the temperature is raised to 95°C at 1.5°C/min, and the viscosity is measured when the temperature reaches 95°C.

In the enzymatic treatment applied to a starch or crosslinked starch, enzyme may act on the starch or crosslinked starch under pH conditions where the enzyme can react, and the enzymatic treatment may be carried out by a known method depending on the type of enzyme used. The types of enzyme used for the enzymatic treatment are as described in the column "Processing characteristics".

The concentration of starch or crosslinked starch during enzymatic treatment is not particularly limited as long as the enzyme reaction is possible. For example, the concentration of starch or crosslinked starch at the start of the enzymatic treatment is 10 to 45% by weight, preferably 20 to 45% by weight, more preferably 30 to 45% by weight.

The concentration of enzyme during the enzymatic treatment is not particularly limited as long as the enzyme reaction is possible, and may be appropriately set according to the degree of enzymatic treatment to be applied, the activity of enzyme used, the reaction temperature, the reaction time, etc. For example, the concentration of enzyme added at the start of the enzymatic treatment is 0.05 to 5.0% by weight, preferably 0.1 to 3.0% by weight, more preferably 0.2 to 1.0% by weight. The concentration of enzyme may be sufficient to allow the enzyme reaction to proceed. If the concentration is within the above range, sufficient progress of the enzyme reaction can be observed, and therefore, there is no need to examine the enzyme activity (number of units) in detail.

The pH during the enzymatic treatment is not particularly limited as long as the enzyme reaction is possible, may be appropriately set according to the type of enzyme used, and is, for example, pH 3.5 to 5.0, preferably pH 3.7 to 4.7, more preferably pH 4.0 to 4.5.

The temperature during the enzymatic treatment is not particularly limited as long as the enzyme reaction is possible and the temperature is in a range where starch or crosslinked starch is not gelatinized, may be appropriately set according to the type of enzyme used, and is, for example, 40 to 60°C, preferably 42 to 55°C, more preferably 45 to 50°C.

The reaction time of the enzymatic treatment may be appropriately set according to the degree of enzymatic treatment to be applied, the activity of the enzyme used, the reaction temperature, etc., and is, for example, 1 to 16 hours, preferably 1 to 8 hours, more preferably 1 to 4 hours.

The degree of the enzymatic treatment is not particularly limited, but from the viewpoint of suitably producing a pregelatinized starch that satisfies the degree of cold water swelling and the mixograph characteristics, the amount of saccharide cleaved and eluted by the enzymatic treatment may be set to 3 to 40 parts by weight, preferably 5 to 30 parts by weight, more preferably 10 to 20 parts by weight per 100 parts by weight of starch or crosslinked starch subjected to the enzymatic treatment.

In the step 1, when starch is modified in the order of crosslinking treatment and enzymatic treatment, the dispersion after crosslinking treatment may be subjected to enzymatic treatment as it is, and after the pH of the dispersion after crosslinking treatment may be adjusted as necessary, the dispersion may be subjected to enzymatic treatment. Furthermore, the dispersion after crosslinking treatment may be concentrated and, if necessary, subjected to the enzymatic treatment after the pH is adjusted, and crosslinked starch may be recovered from the dispersion after the crosslinking treatment and, if necessary, washed and then subjected to enzymatic treatment. After the enzymatic treatment, the enzyme contained in the dispersion after the enzymatic treatment may be deactivated as necessary.

When starch is modified in the order of the enzymatic treatment and the crosslinking treatment in the step 1, the dispersion after the enzymatic treatment may be subjected to the crosslinking treatment as it is or after the pH is adjusted. Alternatively, after enzyme contained in the dispersion after the enzymatic treatment may be deactivated, the pH may be adjusted as necessary to be subjected to the crosslinking treatment. Further, after the enzyme contained in the dispersion after the enzymatic treatment may be deactivated and/or the dispersion may be concentrated, the pH may be adjusted as necessary to be subjected to the crosslinking treatment. Furthermore, the enzyme-treated starch may be recovered from the dispersion after the enzymatic treatment, washed as necessary, and then subjected to the enzymatic treatment.

The enzyme-treated crosslinked starch obtained in the step 1 is subjected to the step 2 described later. The dispersion after completion of the step 1 may be subjected to the step 2 as it is, or the dispersion after completion of the step 1 may be concentrated and subjected to the step 2. The enzyme-treated crosslinked starch may be recovered from the dispersion after the completion of the step 1, washed as necessary, and then subjected to the step 2.

### [Step 2]

In the step 2, the enzyme-treated crosslinked starch obtained in the step 1 is pregelatinized.

The pregelatinization of the enzyme-treated crosslinked starch may be carried out by a known method. In the pregelatinization of the enzyme-treated crosslinked starch, starch may be heated at a gelatinization start temperature or higher while the starch is dispersed in water, and may be dried.

Specific examples of the pregelatinization of the enzyme-treated crosslinked starch include a method of, while dispersing the enzyme-treated crosslinked starch in water at a concentration of 10 to 50% by weight, preferably 20 to 45% by weight, more preferably 30 to 40% by weight, heating the dispersion at a temperature of 80 to 500°C, preferably 85 to 450°C, more preferably 90 to 400°C to semi-gelatinize or gelatinize the starch and thus to dry the semi-gelatinized or gelatinized starch.

Although the heating method is not particularly limited, in case of general industrial production, examples of the heating method include a continuous production method with a drum dryer or an extruder. When a drum dryer or an extruder is used, heating is performed at a high temperature, so that there is an advantage that a drying step is not generally required. The semi-gelatinized or gelatinized starch may be dried by for example, blow drying, spray drying, freeze drying, and so on.

As the step 2, a pregelatinizing treatment using drum dryer is suitable from the viewpoint of producing a pregelatinized starch which suitably has the degree of cold water swelling and the mixograph characteristics and can more favorably achieve both suppression of formation of lumps and expression of sufficient viscosity. Examples of suitable conditions for the pregelatinizing treatment using drum drying include a condition where while the enzyme-treated crosslinked starch is dispersed in water at a concentration of 20 to 45% by weight, preferably 30 to 45% by weight, more preferably 40 to 45% by weight, treatment is performed at a set temperature of 120 to 150°C, preferably 125 to 145°C, more preferably 130 to 140°C.

The pregelatinized enzyme-treated crosslinked starch obtained in the step 2 has the degree of cold water swelling and the mixograph characteristics, and can be used as it is as the pregelatinized starch of the present invention. Further, the pregelatinized enzyme-treated crosslinked starch obtained in the step 2 may be subjected to a pulverization treatment, a sizing treatment, or the like, if necessary. Furthermore, the obtained pregelatinized enzyme-treated crosslinked starch may be further subjected to processing (chemical modification treatment) or the like, if necessary.

### Intnted use

The pregelatinized starch of the present invention is used as an ingredient for various foods. Since the pregelatinized starch of the present invention can be rapidly dispersed while suppressing formation of lumps upon addition of water thereto and can provide a sufficient viscosity, processing of food is facilitated, and the flavor and texture of food can be improved.

The type of food containing the pregelatinized starch of the present invention is not particularly limited and may be any food using conventional pregelatinized starch, and examples include gel-like foods, pasty foods, and so on.

In the present invention, the term "gel-like foods" refer not only to foods that are in the form of a gel as a whole, but also to foods containing a gel which is temporarily formed during production process and whose moisture has been reduced by baking or the like (for example, bakery products). The type of gel-like foods is not particularly limited, and examples include noodles such as udon, soba, hiyamugi, soumen, Chinese noodles, pasta, spaghetti, and macaroni; bakery products such as bread, pizza dough, pie dough, ice cream cone cup, monaka shell, and cream puff shell; confectionaries such as jelly, mousse, pudding, yogurt, mizu-manju, kuzumochi, and uirou; fish paste products such as kamaboko, fish sausage, fish ham, minced fish, chikuwa, hanpen, and satsumaage; baked confectioneries such as biscuits, cookies, crackers, okaki, rice crackers, and puffed snacks; western confectioneries such as sponge cakes, chiffon cakes, castella, madeleine, financiers, pound cakes, roll cakes, cake donuts, and yeast donuts; and Chinese food preparations such as Chinese steamed bun (chuka-man) skin and dumpling (gyoza) skin.

The type of pasty foods is not particularly limited, and examples include creams such as custard cream, whipped cream, and sour cream; frozen confectionery such as soft cream; salsa and sauces such as meat sauce, white sauce, demiglace sauce, salsa for split and broiled fish, and glaze for mitarashi dango; flour paste, plant (vegetables, potatoes, fruits, etc.) paste, cheese paste, liquid food, baby food, mayonnaise, dressings, mustard, wasabi paste, and garlic paste.

Suitable examples of foods containing the pregelatinized starch of the present invention include flour processed food. The flour processed food is food processed from wheat flour. Specific examples of flour processed food include those in which wheat flour is used among the gel-like foods described above. The pregelatinized starch of the present invention has a low water absorption rate and has a property of hardly causing lumps, so that when the pregelatinized starch is used in flour processed food, it is difficult to inhibit water absorption of wheat flour during production. As a result, gluten in the wheat flour is sufficiently formed. Even if the amount of water added is increased, the dough is not sticky, and workability is improved. In particular, when the pregelatinized starch of the present invention is used for bakery products, the pregelatinized starch provides a sufficient viscosity, so that the bakery products have water retention and can have a fluffy and soft texture. For example, in the case of Baguette, the structure of crumb is large, and effects such as a remarkable improvement in keeping quality can be provided, and in the case of Danish bread, effects such as an increase in volume can be provided. In particular, when the gelatinized starch of the present invention and gluten are used in combination in a bakery food, the above effects can be further improved and provided. In particular, in a case where the bakery food is a sandwich, when the pregelatinized starch of the present invention and gluten are used in combination, it is possible to effectively suppress moisture of ingredients from transferring to bread. In the present invention, gluten includes raw gluten, flash dried gluten and the like, and further includes gluten treated with an acid, alkali, enzyme, oxidizing agent, reducing agent and the like and then dried. Gluten may contain auxiliary materials, such as fats and oils and an emulsifier. When the pregelatinized starch of the present invention and gluten are used in combination in a bakery food, the ratio of the two is not particularly limited. For example, the amount of gluten is 10 to 500 parts by weight, preferably 15 to 250 parts by weight, more preferably 20 to 150 parts by weight per 100 parts by weight of the pregelatinized starch of the present invention.

Other suitable examples of foods containing the pregelatinized starch of the present invention include fish paste products. Since the pregelatinized starch of the present invention has a property of hardly causing lumps, when the pregelatinized starch is used in a fish paste product, the pregelatinized starch can be dissolved in water and add to kneaded dough during production. Furthermore, since the pregelatinized starch of the present invention provides a sufficient viscosity, even if the amount of fish meat used is reduced by 10 to 30%, kneaded dough can have a suitable viscosity without changing the texture.

In a case of Chinese steamed bun skin containing the pregelatinized starch of the present invention, the dough is not sticky even if the amount of water added is increased, moldability is good, and workability is improved. In addition, moist, airy, and soft texture can be given, and a tensioned surface can be formed. Furthermore, the Chinese steamed bun skin containing the pregelatinized starch of the present invention does not harden even when heated in a microwave oven and can maintain a good texture. In particular, when the pregelatinized starch of the present invention and gluten are used in combination in Chinese steamed bun skin, it is also possible to effectively suppress moisture transfer to the Chinese steamed bun skin after hot bender. When the pregelatinized starch of the present invention and gluten are used in combination in Chinese steamed bun skin, the ratio of the two is not particularly limited. For example, the amount of gluten is 10 to 500 parts by weight, preferably 15 to 300 parts by weight, more preferably 25 to 200 parts by weight per 100 parts by weight of the pregelatinized starch of the present invention.

In a case of noodles containing the pregelatinized starch of the present invention, the effect of preventing softening with hot water or by boiling and the quick boiling effect can be imparted, and even when heated in a microwave oven, the noodles can be quickly boiled while preventing softening with hot water or by boiling.

In a case of a pasty food containing the pregelatinized starch of the present invention, the pasty food can be dispersed without forming lumps due to the pregelatinized starch, and it is also possible to express a highly shape-retaining viscosity. Furthermore, the pasty food containing the pregelatinized starch of the present invention has good flavor release. In particular, in a case of pasty food in which solid content is dispersed, as in wasabi paste and the like, dispersibility of the solid content such as wasabi is also improved, and operability during production is further improved.

The amount of the pregelatinized starch of the present invention in the foods containing the pregelatinized starch is not particularly limited and may be the same as the amount of a pregelatinized starch conventionally used in the foods, and while a portion thereof may be used in the conventional pregelatinized starch, the remaining portion may be replaced with the pregelatinized starch of the present invention.

Foods containing the pregelatinized starch of the present invention can be produced by a known method in the art depending on the type of food.

### EXAMPLES

The present invention will next be described in more detail by way of examples, which should not be construed as limiting the invention thereto.

### 1. Production of pregelatinized starch

### [Example 1]

A tapioca starch was subjected to the following phosphate-crosslinking treatment, enzymatic treatment, and pregelatinizing treatment to obtain a pregelatinized enzyme-treated distarch phosphate.

### (Phosphate-crosslinking treatment)

A tapioca starch was dispersed in water at 40% by weight to prepare a starch dispersion. Next, sodium chloride was added to the starch dispersion at 0.8% by weight. Thereafter, the mixture was heated to 42°C, and 3.75% by weight aqueous sodium hydroxide solution was added dropwise, and the pH is adjusted to pH 11.3. While the pH was maintained, sodium trimetaphosphate was added at 0.12% by weight and reacted for 6 hours, thus producing a distarch phosphate. When the distarch phosphate obtained by the phosphate-crosslinking treatment was subjected to amylogram measurement under the above-mentioned conditions, the amylo viscosity was 121 BU.

### (Enzymatic treatment)

The dispersion after the phosphate-crosslinking treatment was heated to 45°C, and the pH was adjusted to pH 4.2 using a 25% by weight sulfuric acid aqueous solution. Next, while the dispersion was heated to 45°C, glucoamylase derived from Aspergillus niger (manufactured by OPTIDEX-L400 GENENCOR) was added to 0.04% by weight, and reacted for 4 hours, whereby an enzyme-treated distarch phosphate was produced. In the enzymatic treatment, 10 parts by weight of saccharide was eluted per 100 parts by weight of the distarch phosphate.

### (Pregelatinizing treatment)

The pH of the dispersion after the enzymatic treatment was adjusted to pH 5.5, the dispersion was thoroughly washed and dehydrated, and an enzyme-treated distarch phosphate was recovered. Next, the enzyme-treated distarch phosphate was dispersed in water at 45% by weight, and dried by heating with a drum dryer to obtain a pregelatinized enzyme-treated distarch phosphate. The drying by heating with a drum dryer used a double drum dryer with a diameter of 2 m and a width of 1 m, and the drying was carried out by operating the dryer while rotating the dryer at 8 rpm under conditions where a supply amount of the pregelatinized enzyme-treated distarch phosphate was 450 Kg/, the set temperature was 130°C, and the treatment time was 2 hours.

The obtained pregelatinized enzyme-treated distarch phosphate was pulverized and subjected to the test described below.

### [Example 2]

A pregelatinized enzyme-treated distarch phosphate was obtained under the same conditions as in Example 1 except that sodium trimetaphosphate was added at 0.28% by weight during the phosphate-crosslinking treatment. When the distarch phosphate obtained after the phosphate-crosslinking treatment was subjected to amylogram measurement under the above-mentioned conditions, the amylo viscosity was 13 BU. The obtained pregelatinized enzyme-treated distarch phosphate was pulverized and subjected to the test described below.

### [Comparative Example 1]

A tapioca starch (unmodified state) was dispersed in water at 45% by weight, and dried by heating with a drum dryer to obtain a pregelatinized starch. The conditions for the drying by heating with a drum dryer were the same as in Example 1 except that the set temperature was 150°C. The obtained pregelatinized starch was pulverized and subjected to the test described below.

### [Comparative Example 2]

A tapioca starch was dispersed in water at 40% by weight to prepare a starch dispersion. Next, the enzymatic treatment was carried out under the same conditions as in Example 1 to produce an enzyme-treated starch. Next, the pregelatinizing treatment was carried out under the same conditions as in Example 1 except that the set temperature of the drum dryer was 150°C to obtain an enzyme-treated gelatinized starch. The obtained enzyme-treated gelatinized starch was pulverized and subjected to the test described below.

### [Comparative Example 3]

A phosphate-crosslinking treatment was carried out under the same conditions as in Example 1 except that sodium trimetaphosphate was added at 0.28% by weight during the phosphate-crosslinking treatment to produce a distarch phosphate. Next, the pregelatinizing treatment was carried out under the same conditions as in Example 1 to obtain a pregelatinized distarch phosphate. The obtained pregelatinized distarch phosphate was pulverized and subjected to the test described below.

### [Comparative Example 4]

A pregelatinized starch was obtained under the same conditions as in Comparative Example 1 except that the set temperature was changed to 70°C. The obtained pregelatinized starch was pulverized and subjected to the test described below.

### 2.Evaluation of pregelatinized starch

### 2-1.Evaluation method

### (Degree of cold water swelling)

1 g in terms of dry weight of a pregelatinized starch was dispersed in 100 ml of water at 25°C contained in a 200 ml beaker. Next, the mixture was stirred for 30 minutes using a stirrer bar at 500 rpm (2 Mag magnetic motion) in a thermostatic bath (TAITEC THEAMO MINDER) at 25°C. Thereafter, the solution was transferred to two 50 ml centrifuge tubes, centrifuged (3000 rpm, 10 minutes: H-26/Rotor RF110 manufactured by KOKUSAN), and separated into a gel layer and a supernatant layer. The gel layer was then weighed and taken as A. Furthermore, the gel layer was dried to a solid state (of constant weight at 105°C), and the weight of the resulting solid was measured and taken as B. A/B was calculated to determine the degree of cold water swelling.

### (Mixograph characteristics)

25 ml of water was added to a mixture of 5 g of pregelatinized starch and 25 g of strong flour, and using a mixogram (35-g Mixograph, National Manufacturing Division, TMCO, USA: No. 10 spring), according to AACC (American Association of Cereal Chemists) method 54-40.02 established by AACC, a change in torque (torque%) was measured for 100 seconds at a temperature of 15°C by a mixograph test method.

The middle line (moving average line) was obtained by averaging the amplitude of the torque (torque%) appearing in the measurement. The middle line (moving average line) was obtained by using software of MIX SMARTver2.0.590 (manufactured by National Cereal Chemistry Equipment) and setting Midcurve filter to 80, No.Stages to 2, Mid slope window (%) to 7.5, and Mid peak fit window (%) to 5.

### (Viscosity)

While 180 ml of water at 25°C contained in a 200 ml beaker was stirred at 1200 rpm with a stirrer, 1 g in terms of dry weight of a pregelatinized starch was quickly added and stirred for 1 minute. Thereafter, using a B-type viscometer (VISCOMETER TV-20, manufactured by Toki Sangyo Co., Ltd.), viscosity was measured under conditions where a No. 5 rotor, 25°C, rotation speed of 30 rpm, and after an elapse of 1 minute.

### (State of formation of lumps)

While 180 ml of water at 25°C contained in a 200 ml beaker was stirred at 1200 rpm with a stirrer, 7 g in terms of dry weight of a pregelatinized starch was quickly added, and stirring was continued for 1 minute. Thereafter, the state of formation of lumps was visually observed and evaluated according to the following criteria.

### • Criteria for judgment in state of formation of lumps

⊙: No lump is observed at all, and uniform dispersion is obtained.
○: About 1 to 2 small lumps are observed.
×: Several large lumps are observed.
××: Almost no dispersion is observed, and starch exists as large lumps on the surface.

### 2-2. Evaluation Results

For each pregelatinized starch, a graph showing the consistency (torque%) (calculated from the middle line) for each measurement time in the mixograph test method is shown in Figs. 1 and 2. Table 1 shows evaluation results of each pregelatinized starch.

As a result, the pregelatinized enzyme-treated distarch phosphates of Examples 1 and 2 had a degree of cold water swelling of 7 to 10, and as the mixograph characteristics, Y(t) ≤ Y(t+0.1) was satisfied in all range where t was 0 to 99.9 seconds. Those pregelatinized starches could be rapidly dispersed while suppressing formation of lumps upon addition of water thereto and could provide a sufficient viscosity.

On the other hand, in the pregelatinized starch of Comparative Example 1, the enzyme-treated pregelatinized starch of Comparative Example 2, and the pregelatinized distarch phosphate of Comparative Example 3, the degree of cold water swelling exceeded 10, and as the mixograph characteristics, Y(t) ≤ Y(t + 0.1) was not satisfied in all range where t was 0 to 99.9 seconds. Although high viscosity could be provided upon addition of water thereto, formation of lumps could not be suppressed. In the pregelatinized starch of Comparative Example 4, as the mixograph characteristics, Y(t) ≤ Y(t + 0.1) was satisfied in all range where t was 0 to 99.9 seconds. However, the degree of cold water swelling was less than 7, and although formation of lumps could be suppressed, it was insufficient in terms of imparting viscosity.

**[Table 1]**

| | Degree of cold water swelling | Mixograph characteristics^{#} | Viscosity (mPa·s) | State of formation of lumps | Comprehensive evaluation | |
|---|---|---|---|---|---|---|
| Example 1 | 9.76 | Satisfaction | 1820 | ⊙ | Good | No lump is formed, and sufficient viscosity is provided |
| Example 2 | 7.07 | Satisfaction | 1210 | ⊙ | Good | No lump is formed, and sufficient viscosity is provided |
| Comparative Example 1 | 26.43 | Nonsatisfaction | 12910 | ×× | Poor | Lumps are formed |
| Comparative Example 2 | 25.26 | Nonsatisfaction | 13100 | ×× | Poor | Lumps are formed |
| Comparative Example 3 | 7.96 | Nonsatisfaction | 1180 | × | Poor | Lumps are formed |
| Comparative Example 4 | 1.36 | Satisfaction | 0 | ⊙ | Poor | Viscosity cannot be provided |

| | | | | | | |
|---|---|---|---|---|---|---|
| # In the mixograph test method, t is a time (sec) which has passed since measurement initiation, Y(t) is a torque (torque%) after t seconds determined from the middle line, and Y(t+0.1) is a torque (torque%) after t+0.1 seconds determined from the middle line. | | | | | | |

When Y(t) ≤ Y(t+0.1) is satisfied in all range where t is 0 to 99.9 seconds, the term "Satisfaction" is described. When Y(t) ≤ Y(t+0.1) may not be satisfied even a moment in the range where t is 0 to 99.9 seconds, the term "Nonsatisfaction" is described.

### 3. Production and evaluation of bread using pregelatinized starch

Using the dough having the composition shown in Table 2, bread was produced through the steps shown in Table 3.

**[Table 2]**

| Composition of dough | | | |
|---|---|---|---|
| Blended item (g) | Sponge dough | Dough | |
| | | Control | Test product |
| Strong flour (Camellia) | 700 | 300 | 200 |
| Pregelatinized starch of Example or Comparative Example | - | - | 100 |
| Granulated sugar | 50 | 250 | 250 |
| Oil and fat (shortening) | 200 | 100 | 100 |
| Egg yolk | 100 | - | - |
| Raw yeast (Kaneka Yeast GA) | 30 | 25 | 25 |
| Yeast food (C Oriental Food) | 1 | - | - |
| Skim milk powder | - | 30 | 30 |
| Emulsifier (Ryoto Sugar Ester S-1570) | 20 | - | - |
| Salt | - | 10 | 10 |
| Water | 280 | 170 | 270 |

**[Table 3]**

| Process for producing bread | |
|---|---|
| <Sponge dough> | |
| Mixing | Low speed 3 minutes → medium speed 5 minutes |
| Kneading temperature | 26°C |
| Fermentation time (27°C, 75%) | 2 hours |

| <Dough> | |
|---|---|
| Mixing (↓ indicates addition of shortening) | Low speed 3 minutes → medium speed 4 minutes → ↓ → medium speed 4 minutes → high speed 4 minutes |
| Kneading temperature | 27°C |
| Floor time (27°C, 75%) | 50 minutes |
| Divided weight | 85 g |
| Bench time (27°C, 75%) | 30 minutes |
| Shaping | |
| Final Proof (32°C, 70%) | 50 minutes |

| <Baking> | |
|---|---|
| Baking: upper and lower 190°C | 15 minutes |

The state of dough during mixing, the texture of bread after 1 day from the production, and the extent of retrogradation of the starch in the bread after 4 days from the production were evaluated according to the following criteria.

### • Criteria for judgment in state of dough during mixing

⊙: No stickiness.
○: Although the dough adheres to the hand, the dough can be removed from the hand without being cut (Control state).
×: The dough is slightly cut and adheres to the hand.
××: The dough cannot be handled by the hand.

### • Criteria for judgment in texture of bread after 1 day from production (sensory evaluation)

⊙: Moist, airy and crisp.
○: Moist and crisp but slightly heavy.
×: Water oozes, the texture is heavy, and poor in meltability in the mouth.
××: Wet, clogged texture, and sticky

### • Criteria for judgment in extent of retrogradation of starch in bread after 4 days from production (sensory evaluation)

⊙: Good texture with almost no change from texture after 1 day from production.
○: Airy but slightly dry.
×: Poor in crispness, and dry
××: Hard, dry and crumbled.

The evaluation results are shown in Table 4. When the pregelatinized starches of Examples 1 and 2 were used, the bread dough was not sticky and was easy to gather, so that operability during production was improved. Furthermore, when the pregelatinized starches of Examples 1 and 2 were used, the texture after 1 day from the production was good. The good texture was maintained even after 4 days from the production, and starch retrogradation could be suppressed. On the other hand, in the pregelatinized starches of Comparative Examples 1 to 4, the state of the bread dough was poor, and the operability during production was low. In addition, these pregelatinized starches were not satisfactory in terms of the texture after 1 day from the production and starch retrogradation after 4 days from the production.

**[Table 4]**

| | State of dough during mixing | Texture of bread after 1 day from production | Extent of retrogradation of starch in bread after 4 days from production |
|---|---|---|---|
| Example 1 | ○ | ⊙ | ⊙ |
| Example 2 | ⊙ | ⊙ | ⊙ |
| Comparative Example 1 | ×× | ×× | × |
| Comparative Example 2 | ×× | ×× | × |
| Comparative Example 3 | × | × | × |
| Comparative Example 4 | ×× | Δ | ×× |

### 4. Production and evaluation of sandwich using pregelatinized starch and gluten

Using the dough having the composition shown in Table 5, a sandwich was produced through the steps shown in Table 6.

**[Table 5]**

| Composition of dough | | | | | |
|---|---|---|---|---|---|
| Blended item (g) | Sponge dough | Dough | | | |
| | | Control | Test product 1 | Test product 2 | Comparative test product 1 |
| Strong flour (Ocean) | 700 | 300 | 260 | 280 | 280 |
| Pregelatinized starch of Example 1 | - | - | 20 | 20 | - |
| Gluten (A-Glu SS: manufactured by Glico nutrition Co., Ltd.) | - | | 20 | - | 20 |
| Caster sugar | - | 60 | 60 | 60 | 60 |
| Oil and fat (shortening) | - | 50 | 50 | 50 | 50 |
| Raw yeast (Kaneka Yeast GA) | 25 | - | - | - | - |
| Yeast food (C Oriental Food) | 1 | - | - | - | - |
| Skim milk powder | - | 30 | 30 | 30 | 30 |
| Salt | - | 20 | 20 | 20 | 20 |
| Water | 400 | 280 | 320 | 300 | 300 |

**[Table 6]**

| Process for producing sandwich | |
|---|---|
| <Sponge dough> | |
| Mixing | Low speed 2 minutes → medium speed 2 minutes |
| Kneading temperature | 26°C |
| Fermentation time (27°C, 75%) | 2.5 hours |

| <Dough> | |
|---|---|
| Mixing (↓ indicates addition of shortening) | Low speed 3 minutes → medium speed 3 minutes → ↓ → low speed 2 minutes → medium speed 3 minutes → medium speed 3 minutes |
| Kneading temperature | 27°C |
| Floor time (27°C, 75%) | 20 minutes |
| Divided weight | 245 g × 3 |
| Bench time (27°C, 75%) | 30 minutes |
| Shaping/mold packing | U shape, 1.5 loaf |
| Final Proof (32°C, 70%) | 50 minutes |

| <Baking> | |
|---|---|
| Baking: upper 220°C, lower 230°C | 35 minutes |

| <Preparation of sandwich> | |
|---|---|
| Slice width | Cut crust and slice into 16 mm |
| Sandwiching of ingredients | Sandwich 50 g of egg filling between two breads |

The texture of a sandwich stood still for 2 hours without being wrapped after slicing and moisture transfer from ingredients to bread after 24 hours from sandwiching of the ingredients were evaluated according to the following criteria.

### • Criteria for judgment in texture of sandwich after slicing (sensory evaluation)

⊙: Moist, airy and crisp.
○: Moist but poor-crisp.
×: No moist feeling and poor-crisp.
××: There is dry feeling, and the surface is dry.

### • Criteria for judgment in moisture transfer (sensory evaluation)

⊙: Moisture of the ingredients does not penetrate into the bread.
○: Moisture of the ingredients slightly penetrates into the bread.
×: Moisture of the ingredients penetrates into the bread, but does not reach the outside of the bread.
××: Moisture of the ingredients penetrates into the bread and reaches the outside of the bread.

The evaluation results are shown in Table 7. When the pregelatinized starch of Example 1 was blended (Test products 1 and 2), the operability during production was improved, and a more excellent texture was recognized. In particular, when the pregelatinized starch of Example 1 and gluten were used in combination (Test product 1), the transfer of moisture from the ingredients to the bread could be sufficiently suppressed. On the other hand, when the pregelatinized starch was not blended and only gluten was blended (Comparative test product 1), although the transfer of moisture from the ingredients to the bread could be suppressed, drying after slicing progressed, and the product was not satisfactory in terms of the texture.

**[Table 7]**

| | Texture after slicing | Moisture transfer |
|---|---|---|
| Control | ×× | ×× |
| Test product 1 | ⊙ | ⊙ |
| Test product 2 | ○ | ×× |
| Comparative test product 1 | × | ○ |

### 5. Production and evaluation of Chinese steamed bun using pregelatinized starch and gluten

Using the dough having the composition shown in Table 8, Chinese steamed bun was produced through the steps shown in Table 9.

**[Table 8]**

| Blended item (g) | Sponge dough | Dough | | | |
|---|---|---|---|---|---|
| | | Control | Test product 3 | Test product 4 | Comparative test product 2 |
| Strong flour (Ocean) | 700 | - | - | - | - |
| Wheat flour (Toku Suzume) | | 300 | 300 | 300 | 300 |
| Pregelatinized starch of Example 1 | - | - | 20 | 20 | - |
| Gluten (A-Glu SS: manufactured by Glico nutrition Co., Ltd.) | - | | 20 | - | 20 |
| Caster sugar | - | 230 | 60 | 60 | 60 |
| Oil and fat (lard) | - | 50 | 50 | 50 | 50 |
| Raw yeast (Kaneka Yeast GA) | 15 | - | - | - | - |
| Skim milk powder | - | 30 | 30 | 30 | 30 |
| Water | 350 | 90 | 90 | 90 | 90 |

**[Table 9]**

| Process for producing Chinese steamed bun | |
|---|---|
| <Sponge dough> | |
| Mixing | Low speed 2 minutes → medium speed 4 minutes |
| Kneading temperature | 25°C |
| Fermentation time (27°C, 70%) | 1 hour |

| <Dough> | |
|---|---|
| Mixing (↓ indicates addition of shortening) | ↓ low speed 3 minutes → medium speed 3 minutes → high speed 1 minute |
| Kneading temperature | 27°C |
| Floor time (27°C, 70%) | 10 minutes |
| Divided weight | 60 g (filling 40) |
| Bench time (27°C, 70%) | 10 minutes |
| Enclosure | U shape, 1.5 loaf |
| Final Proof (40°C, 50%) | 30 minutes |

| <Steaming> | |
|---|---|
| Steaming: 0.2 atmosphere | 12 minutes |

| <Freezing> | |
|---|---|
| Freezing: -40°C | 30 minutes |

The texture of Chinese steamed bun after the frozen Chinese steamed bun was heated for 2 minutes in a microwave oven (600W) and moisture transfer to Chinese steamed bun skin after heating for 8 hours in a hot bender (80°C) were evaluated according to the following criteria.

### • Criteria for judgment in texture of after heating in microwave oven (sensory evaluation)

⊙: Moist and airy.
○: Chinese steamed bun is moist but slightly becomes a clump during chewing.
×: Airy but hard.
××: There is dry feeling, and it is hard.

### • Criteria for judgment in moisture transfer to Chinese steamed bun skin after hot bender (sensory evaluation)

⊙: Vapor moisture does not penetrate into skin of Chinese steamed bun.
○: Vapor moisture slightly penetrates into skin of Chinese steamed bun.
×: Although vapor moisture penetrates into skin of Chinese steamed bun, the shape of the Chinese steamed bun is retained.
××: Vapor moisture penetrates into skin of Chinese steamed bun, the shape of the Chinese steamed bun is collapsed.

The evaluation results are shown in Table 10. When the pregelatinized starch of Example 1 was blended (Test products 3 and 4), the operability during production was improved, and a more excellent texture was recognized. In particular, when the pregelatinized starch of Example 1 and gluten were used in combination (Test product 3), the moisture transfer to the skin of Chinese steamed bun after hot bender could be sufficiently suppressed. On the other hand, when the pregelatinized starch was not blended and only gluten was blended (Comparative test product 2), although the moisture transfer to the skin of Chinese steamed bun after hot bender could be suppressed, drying after heating in a microwave oven progressed, and the product was not satisfactory in terms of the texture.

**[Table 10]**

| | Texture after heating in microwave oven | Moisture transfer to skin of Chinese steamed bun after hot bender |
|---|---|---|
| Control | ×× | ×× |
| Test product 3 | ⊙ | ⊙ |
| Test product 4 | ○ | × |
| Comparative test product 2 | × | ○ |

## Claims

1. A pregelatinized starch having a degree of cold water swelling of 7 to 10,
a middle line (moving average line) for torque (torque%) being determined by the following mixograph test method, and when t is a time (sec) which has passed since measurement initiation, Y(t) is a torque (torque%) after t seconds determined from the middle line, and Y(t + 0.1) is a consistency (torque%) after t + 0.1 seconds determined from the middle line, Y(t) ≤ Y(t + 0.1) being satisfied in all range where t is 0 to 99.9 seconds:
<Mixograph test method>
25 ml of water is added to a mixture of 5 g of target pregelatinized starch and 25 g of strong flour, and according to AACC method 54-40.02 established by AACC (American Association of Cereal Chemists), a change in torque (torque%) is measured for 100 seconds at a temperature of 15°C by the mixograph test method.

2. The pregelatinized starch according to claim 1, wherein a viscosity measured under the following conditions is 1000 mPa·s or more:
<Conditions for measuring viscosity>
While 180 ml of water at 25°C contained in a 200 ml beaker is stirred at 1200 rpm with a stirrer, 1 g in terms of dry weight of a pregelatinized starch to be measured is quickly added and stirred for 1 minute; thereafter, using a B-type viscometer (VISCOMETER TV-20, manufactured by Toki Sangyo Co., Ltd.), viscosity is measured under conditions where a No. 5 rotor, 25°C, rotation speed of 30 rpm, and after an elapse of 1 minute.

3. The pregelatinized starch according to claim 1 or 2, wherein the pregelatinized starch is a modified pregelatinized starch subjected to a crosslinking treatment and an enzymatic treatment.

4. A method for producing a pregelatinized starch comprising:
a step 1 of applying a crosslinking treatment and an enzymatic treatment to a starch to obtain an enzyme-treated crosslinked starch; and
a step 2 of applying a pregelatinizing treatment to the enzyme-treated crosslinked starch obtained in the step 1 to obtain a pregelatinized enzyme-treated crosslinked starch.

5. The method for producing a pregelatinized starch according to claim 4, wherein the crosslinking treatment in the step 1 is a phosphate-crosslinking treatment.

6. The method for producing a pregelatinized starch according to claim 4 or 5, wherein the enzymatic treatment in the step 1 is a treatment using an amylolytic enzyme.

7. The method for producing a pregelatinized starch according to any one of claims 4 to 6, wherein the enzymatic treatment is applied after the crosslinking treatment is applied in the step 1.

8. The method for producing a pregelatinized starch according to any one of claims 4 to 7, wherein the pregelatinizing treatment in the step 2 is a pregelatinizing treatment using a drum dryer.
